# EUROPEAN PATENT APPLICATION

(11) **EP 4 847 009 A1**
(43) Date of publication of application: **02.12.2026**
(21) Application number: 26180860.4
(22) Date of filing: 25.05.2026
(51) Int. Cl.: A61K 8/49

(54) **USE OF A DERMOCOSMETIC FORMULATION COMPRISING EXOSOMES**

(30) Priority: 26.05.2025 IT 202500012019
(71) Applicant: Istituto Ganassini SpA di Ricerche Biochimiche, 20139 Milano (MI) (IT)
(72) Inventor: TERNO, Massimo, 20139 Milano (MI) (IT)
(74) Representative: Ripamonti, Enrico

(57) **Abstract**

Use of a dermocosmetic composition in the field of dermocosmetics, wherein said dermocosmetic composition comprises the ingredients:

i) exosomes, preferably from Lactobacillus species;

ii) Centella asiatica extract, wherein said Centella asiatica extract is further supplemented with cyclic terpenes selected from the group consisting of: madecassoside, asiaticoside, and/or combinations thereof, wherein said cyclic terpenes are obtained from Centella asiatica extract, where said ingredients i) and ii) are present in a range of 0.0001-5% weight/weight (w/w) and said range relates to the amount of madecassoside, or asiaticoside, or to their combination alone, together with the amount of Centella asiatica extract already containing said compound inherently,

and wherein said dermocosmetic use is to counteract cellular senescence.

## Description

### BACKGROUND

Cellular senescence is an irreversible process that halts cell proliferation in response to various stimuli, including oxidative stress, DNA damage, epigenetic alterations, and mitochondrial dysfunction. The accumulation of senescent cells contributes to aging and changes in the skin surface. Lopez-Otin et al. (2023) updated the model of the hallmarks of aging, identifying twelve key characteristics that regulate cellular and systemic decline. Among these, cellular senescence, epigenetic alterations, mitochondrial dysfunction, and chronic inflammation are key factors in the progressive loss of tissue function.

These new insights have spurred research in the fields of dermocosmetics and regenerative medicine, with the aim of identifying innovative strategies to combat skin aging and improve skin health.

One of the most significant aspects of cellular senescence is the development of the SASP (Senescence-Associated Secretory Phenotype), characterized by the secretion of inflammatory cytokines, growth factors, and matrix metalloproteinases (MMPs). In dermal fibroblasts-cells essential for maintaining the extracellular matrix-the accumulation of this phenotype profoundly alters the skin microenvironment. SASP induces chronic inflammation that accelerates the degradation of the extracellular matrix, reduces collagen and elastin synthesis, and compromises the skin's regenerative capacity, promoting the appearance of wrinkles and loss of firmness.

At the molecular level, DNA damage, oxidative stress, and mitochondrial dysfunction play a key role in triggering cellular senescence. The accumulation of reactive oxygen species (ROS) damages cellular structures and amplifies inflammatory signals through the activation of signaling pathways such as NF-kB and AP-1, promoting a vicious cycle of inflammation and tissue degradation.

Senescent cells also exhibit distinctive morphological characteristics, such as enlargement and flattening, which alter the skin's architecture. From a biomolecular perspective, increased beta-galactosidase (SA-B-gal) activity and overexpression of the p16 protein are among the primary indicators of the senescent state and the progressive loss of the skin tissue's regenerative capacity.

It is therefore of scientific interest to identify new strategies capable of modulating these processes and reducing the effects of skin aging.

In the field of dermocosmetics and regenerative medicine, the use of senotherapy molecules is being explored; these molecules are capable of selectively eliminating senescent cells or modulating the SASP phenotype to reduce inflammation and the degradation of the extracellular matrix.

The goal is to develop treatments capable of preserving skin function, improving its appearance, and slowing the aging process.

Currently, there are no optimized cosmetic formulations capable of modulating cellular senescence by acting on markers such as SA-Beta-gal, p16, and p21, which are able to inhibit and counteract the visible signs of skin aging.

Advantageously, the Applicant has developed a dermocosmet l formulation that synergistically and remarkably modulates cellular senescence, acting both on cellular modulation mechanisms and on skin improvement with effects on wrinkles and facial contour.

### FIGURES

**Figure 1****:** staining of plates with β-galactosidase after stimulation with H₂O₂.
**Figure 2****:** graph (2A) and table (2B) showing SA-βgal activity in samples treated with the product of the present invention and stimulated with H₂O₂.
**FIGURE 3****:** In vitro analysis of the influence of the composition of the present invention on the gene expression of p21 and p16.
**Figures 4****,** **5****, and** **6****:** Representative images obtained using the Antera 3D instrument for the in vivo evaluation of the efficacy of the composition of the present invention in a cream formulation.
**Figures 7****,** **8****, and** **9****:** Representative images obtained using the Antera 3D instrument for the in vivo evaluation of the efficacy of the composition of the present invention in a serum formulation.
**Figures 10A****-D:** Photographs of the facial oval of subjects before and after treatment with the product of the present invention in cream form (A and B) and serum form (C and D).
**Figure 11****:** Diagram showing the percentage change in melatonin at various time points.
**Figure 12****:** Brightness data (Skinglossmeter^{®}).

### DETAILED DESCRIPTION OF THE INVENTION

### DEFINITIONS

For the purposes of the present invention, the term "exosomes" refers to nanoscale vesicles secreted by various types of cells, capable of moving freely within our body and penetrating cell membranes, thereby playing an important role as mediators in intercellular communication. Exosomes contain multiple active ingredients (**from growth factors to microRNAs, peptides, and glutathione, a powerful antioxidant)**, key factors that make them unique in the fields of medicine and aesthetic medicine because they are capable of significantly improving the skin regeneration process, restoring the skin barrier, normalizing cell turnover, and, above all, exerting anti-inflammatory and antioxidant effects.

Exosomes can have various origins (human, animal, plant, bacterial).

For the purposes of the present invention, the terms "madecassoside" and "asiaticoside" refer to pentacyclic triterpenoids extracted from the Centella asiatica plant, which possess anti-inflammatory properties.

In a first embodiment, the present invention relates to the use of a dermocosmetic composition in the field of dermocosmetics, wherein said dermocosmetic composition comprises the ingredients:
i) exosomes, preferably from Lactobacillus species;
ii) Centella asiatica extract, wherein said Centella asiatica extract is further supplemented with cyclic terpenes selected from the group consisting of: madecassoside, asiaticoside, and/or combinations thereof,

wherein said cyclic terpenes are obtained from Centella asiatica extract,
where said ingredients i) and ii) are present in a range of 0.0001-5% weight/weight (w/w) and said range relates to the amount of madecassoside, or asiaticoside, or to their combination alone, together with the amount of Centella asiatica extract already containing said compound inherently,
   and
wherein said dermocosmetic use is to counteract cellular senescence.

Preferably, said ingredients i) and ii) are present in a range of 0.001-2% weight/weight (w/w).

Preferably, said Centella asiatica extract is derived from leaves.

The Centella asiatica extract is obtained according to the common technical knowledge of the person skilled in the art. In particular, the Centella asiatica extract of the present invention is obtained so as explained in Example 4.

Preferably, said dermocosmetic composition further comprises one or more ingredients selected from the group consisting of: Water, Isononyl Isononanoate, Methylpropanediol, Pentylene Glycol, Glycerin, Pentaerythrityl Tetraethylhexanoate, Glycereth-26, Niacinamide, Silica, Cetearyl Alcohol, Oryza Sativa (Rice) Bran Oil, Pullulan, Cetearyl Olivate, Butyrospermum parkii (Shea) Butter, Tocopheryl Acetate, Sorbitan Olivate, Sodium Hyaluronate, Chondrus crispus (Carrageenan) Powder, Isoleucine, Leucine, Valine, Lactobacillus Extracellular Vesicles, Butylene Glycol, Acrylates/C10-30 Alkyl Acrylate Crosspolymer, Ammonium Acryloyldimethyltaurate/VP Copolymer, Ethylhexylglycerin, Cetyl Alcohol, Sodium Hydroxide, Tocopherol, Fragrance, and/or mixtures thereof. Preferably, said dermocosmetic composition further comprises one or more ingredients selected from the group consisting of: Water, C9-12 Alkane, Pentylene Glycol, Niacinamide, Isopentyl Diol, Caprylic/Capric Triglyceride, Pullulan, Oryza sativa (Rice) Bran Oil, Panthenol, Silica, Diglycerin, Sodium PCA, Urea, Tocopheryl Acetate, Sodium Hyaluronate, Hydrolyzed Rice Protein, PCA, Allantoin, Isoleucine, Leucine, Lysine, Sorbitol, Valine, Butylene Glycol, Ahnfeltiopsis concinna Extract, Folic Acid, Ferment Filtrate Extract, Magnesium Aspartate, Zinc Gluconate, Copper Gluconate, Lactobacillus Extracellular Vesicles, Sodium Dehydroacetate, 1,2-Hexanediol, Sodium Polyacryloyldimethyl Taurate, Xanthan gum, Pentaerythritol tetra-di-t-butyl hydroxyhydrocinnamate, Polysorbate 80, Tetrasodium glutamate diacetate, Lactic acid, Sodium hydroxide, Sodium benzoate, Potassium sorbate, Perfume (Fragrance), and/or mixtures thereof.

Preferably, the composition of the present invention further comprises one or more ingredients selected from the group consisting of: Water, Glycerin, Methylglucose sesquistearate, Pentaerythrityl tetraethylhexanoate, Pentylene glycol, Niacinamide, Lactobacillus/Punica granatum fruit ferment extract, Oryza sativa (rice) bran oil, Tocopheryl acetate, Radish root/Leuconostoc ferment filtrate, Isoleucine, Leucine, Valine, Lactobacillus extracellular vesicles, Diutan gum (Sphingomonas ferment extract), Butylene glycol, Tetrasodium glutamate diacetate, Lactic acid, Sodium hydroxide, Perfume (fragrance), and/or mixtures thereof.

In another embodiment, the present invention relates to dermocosmetic products comprising the composition for use in the present invention.

Preferably, said dermocosmetic products are selected from the group consisting of: serum, cleanser, cream.

The methods for formulating said products comprising the dermocosmetic composition of the present invention involve technologies known to those skilled in the art, based on their expertise.

Advantageously, the present invention introduces an advancement in the senotherapeutic management of the skin, resolving and compensating for superficial skin alterations resulting from the degradative mechanisms affecting skin cells and their phenotype associated with senescence.

The dermocosmetic composition of the present invention can be used daily to improve the appearance of aging skin. In fact, the composition of the present invention acts advantageously and actively on the superficial layers of the epidermis.

The composition of the present invention effectively reduces the visible signs of aging, with particular reference to:
- wrinkles (volume, area, number, length, depth);
- increased firmness;
- refinement and lifting of the facial contour.

The composition of the present invention advantageously exhibits both short-term and long-term effects in reducing imperfections resulting from aging.

Below are some descriptive, non-limiting examples that demonstrate the advantages of the present invention.

### Bibliographic References

1. Agrawal, R.; Hu, A.; Bollag, W.B. The Skin and Inflamm-Aging. Biology 2023, 12, 1396. https://doi.org/10.3390/biology12111396
2. Ho CY, Dreesen O. Faces of cellular senescence in skin aging. Mech Ageing Dev. September 2021;198:111525. doi: 10.1016/j.mad.2021.111525.
3. Gruber F, Kremslehner C, Eckhart L, Tschachler E. Cell aging and cellular senescence in skin aging - Recent advances in fibroblast and keratinocyte biology. Exp Gerontol. 2020 Feb;130:110780. doi: 10.1016/j.exger.2019.110780. Epub 2019 Nov 30. PMID: 31794850.
4. López-Otín C, Blasco MA, Partridge L, Serrano M, Kroemer G. Hallmarks of aging: An expanding universe. Cell. 2023 Jan 19;186(2):243-278. doi: 10.1016/j.cell.2022.11.001. Epub 2023 Jan 3. PMID: 36599349.
5. Wang AS, Dreesen O. Biomarkers of Cellular Senescence and Skin Aging. Front Genet. 2018 Aug 23;9:247. doi: 10.3389/fgene.2018.00247. PMID: 30190724; PMCID: PMC6115505.
6. Wlaschek M, Maity P, Makrantonaki E, Scharffetter-Kochanek K. Connective Tissue and Fibroblast Senescence in Skin Aging. J Invest Dermatol. 2021 Apr;141(4S):985-992. doi: 10.1016/j.jid.2020.11.010. Epub 2021 Feb 7. PMID: 33563466.
7. Olivieri, F., Procopio, A. D., & Rippo, M. R. (2021). Cellular senescence and senescence-associated secretory phenotype (SASP) in the aging process. Human Aging, 75-88. doi:10.1016/b978-0-12-822569-1.00013-5
8. Gervason S, Napoli M, Dreux-Zhiga A, Lazzarelli C, Garcier S, Briand A, Albouy M, Thepot A, Berthon JY, Filaire E. Attenuation of negative effects of senescence in human- - skin using an extract from Sphingomonas hydrophobicum: development of a new skin care solution. Int J Cosmet Sci. 2019 Aug;41(4):391-397. doi: 10.1111/ics.12534. Epub 2019 Jun 17. PMID: 31032993.

### EXAMPLES

The following examples, which are not exhaustive, were carried out using cream, serum, and cleanser comprising the composition of the present invention, within the ranges indicated.

The microorganisms used for the purpose of the present Examples are *Lactobacillus reuteri.*

### EXAMPLE 1

### IN VITRO EVALUATION OF EFFECTS ON CELLULAR SENESCENCE

The cell line used in the experiment was that of primary fibroblasts, cells found in the dermal layer of the skin. The cells were cultured under completely sterile conditions and maintained in an incubator at 37 °C with 5% carbon dioxide (CO₂).

The components to be tested were dissolved in suitable culture medium. Subsequently, cell viability was analyzed following treatment with the mixtures for 24 hours (MTT assay).

### Cytotoxicity assay (MTT test)

The MTT assay is a colorimetric cytotoxicity assay that allows for the assessment of cell proliferation and viability based on the efficiency of mitochondrial respiration. MTT (3-(4,5-dimethylthiazol-2-yl)-2,5-diphenyl tetrazolium bromide) is a tetrazolium salt that is reduced in viable cells by the action of mitochondrial dehydrogenase. The reduction of MTT leads to the formation of formazan crystals in and subsequent transfer to isopropanol, imparting the characteristic purple staining to the mitochondria of viable cells. Conversely, in stressed or dead cells, which therefore have inactive mitochondria, MTT will not be reduced, resulting in less intense or absent purple staining (Mosman, 1983).

To set up the assay, the cells were seeded homogeneously in a 12-well plate well plate and incubated at 37 °C with 5-10% CO₂. Subsequently, the cells were treated with various combinations of substances previously dissolved in culture medium, starting with the maximum soluble concentration and followed by serial dilutions.

At the end of the treatment (24 h), the cells were examined under an optical microscope to record any morphological changes, and an MTT solution was added to each well for 2 h at 37 °C. The medium was then removed, and the formazan crystals were dissolved with 100 µl of isopropanol. Subsequently, spectrophotometric readings were taken using a microplate reader at a wavelength of 570 nm. Cell viability was calculated by measuring the difference in optical density between each of the tested concentrations compared to the control (untreated cells).

### Assessment of Beta-Galactosidase Levels

The β-galactosidase (Sigma Aldrich) cellular senescence assay is a method based on immunohistochemical staining used to identify cells in the senescence phase.

Senescence is associated with cellular aging, and cells in this phase- -not only lose their ability to divide but also undergo changes in morphology and gene expression profiles. The histochemical staining kit contains all the reagents necessary for the identification of senescent cells and is based on a staining procedure that allows for the assessment of β-galactosidase activity at pH 6. Under these conditions, β-galactosidase activity is easily detectable in senescent cells, appearing as a blue stain.

The cells were seeded in 12-well plates, treated after 24 hours with different concentrations of the test product, and subsequently stained following the kit protocol.

Finally, the cells exhibiting the characteristic bluish staining were counted, and the percentage of cells expressing β-galactosidase (senescent cells) was calculated.

### Results

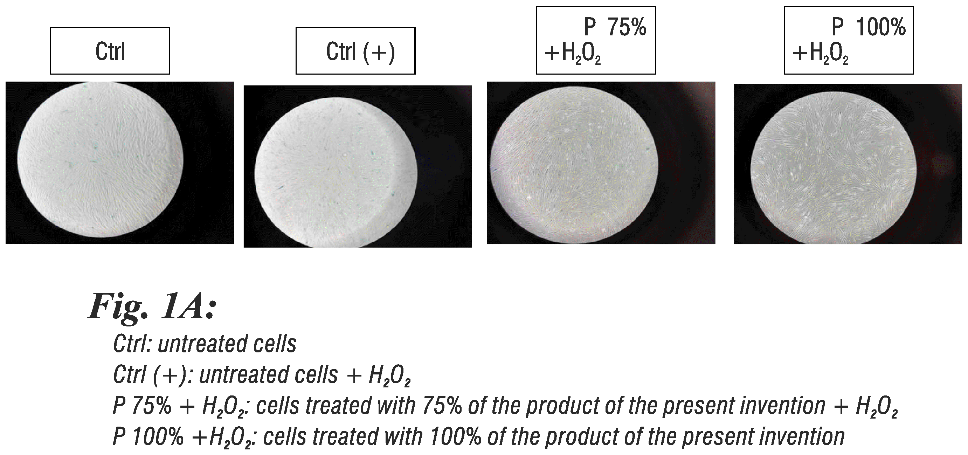

A β-galactosidase staining was performed after stimulation with H₂O₂ to verify whether the composition of the present invention prevents photoaging (Fig. 1).

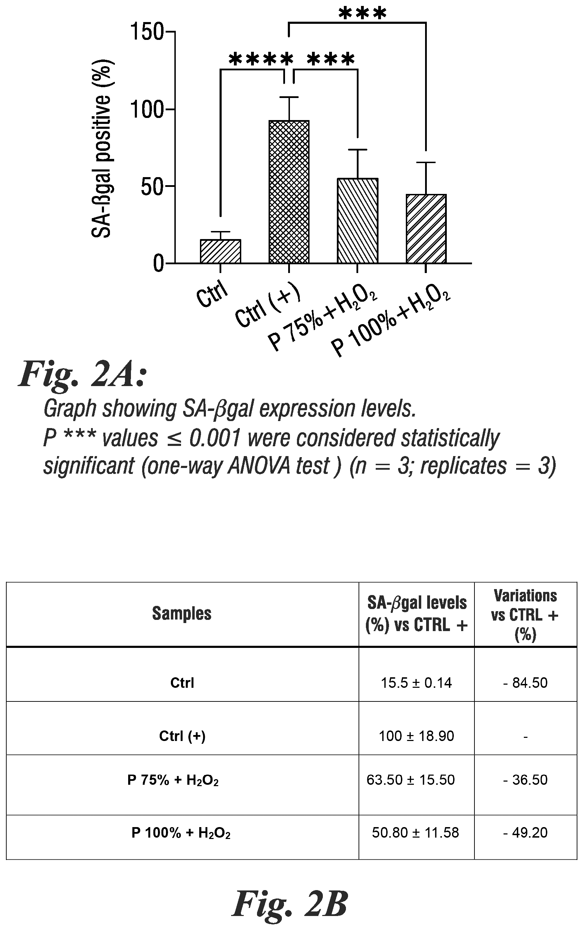

A significant increase in SA-βgal-positive cells (+84.5%) was observed in the control consisting of the untreated sample stimulated with H₂O₂ compared to the untreated sample (Fig. 2B).

Furthermore, the composition of the present invention induced a statistically significant reduction in SA-βgal activity levels of approximately 36.50% and 49.20% following treatment with two selected concentrations of the product of the present invention, 75% and 100%, in a model of stress-induced premature senescence in human fibroblasts.

Figure 2 graphically illustrates SA-βgal activity in samples treated with the product of the present invention and stimulated with H₂O₂. The values indicated that stimulation induces senescence in fibroblasts (+84.50% SA-βgal-positive cells in the positive control sample) and that treatment with the product of the present invention reduces βgal levels by 36.50% and 42.90%, respectively.

Based on the data reported above, the composition of the present invention resulted in a statistically significant reduction in senescence-associated β-galactosidase (sa-β-gal) activity after 24 hours of treatment in an aged model of human fibroblasts.

### Evaluation of p21 and p16 Gene Expression

The samples treated with the mixtures under investigation were analyzed by RT-PCR to assess whether treatment with the products under investigation affects the expression of the genes of interest (p21 and p16, involved in cell cycle arrest).

To perform this test, cells were seeded uniformly in a 12-well plate and incubated at 37 °C in a humidified atmosphere containing 5% CO₂. After 24 hours, the cells were irradiated with a UVB lamp for 72 seconds (75 mJ/cm²) to induce senescence. After 48 hours of recovery, the cells were treated for 24 hours with non-cytotoxic concentrations of the test product.

After 24 hours of treatment, the cells were harvested for RNA extraction using the RNeasy Mini kit (Qiagen). Subsequently, the concentration of total extracted RNA was quantified using MultiskanGO and uDrop Duo Plate, and reverse transcription of total RNA into cDNA was performed using the iScript^{™} Reverse Transcription Supermix kit (Bio-Rad).

Real-time PCR analysis was performed on the target genes p21 and p16; GAPDH (glyceraldehyde-3-phosphate dehydrogenase) was used as a housekeeping gene to normalize gene expression. Reaction conditions included three distinct phases: denaturation of double-stranded cDNA, primer annealing, and extension of the newly synthesized strand; the latter two phases were repeated for 39 consecutive cycles.

The instrument used for analysis was the CFX Connect (Bio-Rad), and the data were analyzed using CFX Maestro software (Bio-Rad).

### Results

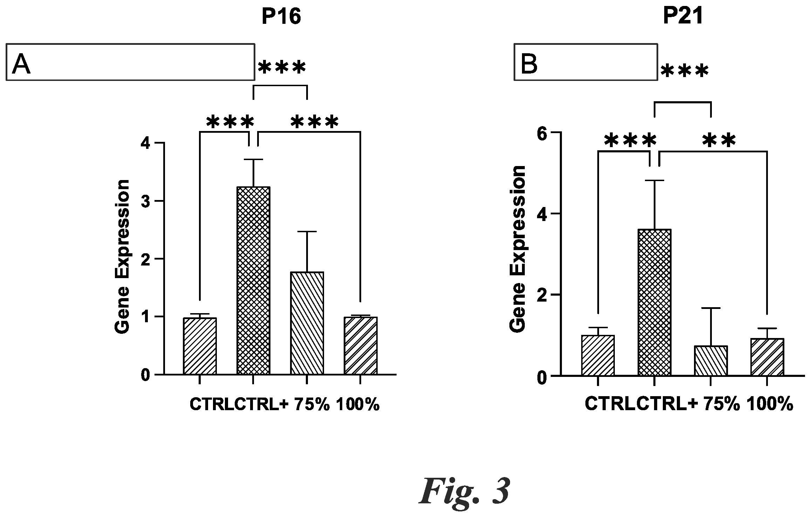

Figure 3 shows the expression of p16 (A) and p21 (B) mRNA after treatment with the test product at the selected concentrations, compared to the positive control (CTRL+ cells, untreated and irradiated with UVB). p ≤ 0.05 (***) is statistically significant compared to the positive control (one-way ANOVA).

The following table shows gene expression data ± standard deviations relative to the positive control sample (untreated cells irradiated with UVB).

| **sample** | **p16 gene expression (increase ± standard deviation)** | **p21 gene expression (increase ± standard deviation)** |
|---|---|---|
| **Ctrl** | 1.00 ± 0.05 | 1.00 ± 0.19 |
| **Ctrl+** | 3.27 ± 0.45 | 3.59 ± 1.62 |
| **75%** | 1.80 ± 0.68 | 1.33 ± 1.53 |
| **100%** | 1.02 ± 0.01 | 1.01 ± 0.14 |

The results obtained show that the composition of the present invention induces a significant reduction in the expression of the p21 and p16 genes after 24 hours of treatment in normal human fibroblasts.

### EXAMPLE 2

### EVALUATION OF THE IN VIVO EFFICACY OF A CREAM, SERUM, AND CLEANSER COMPRISING THE COMPOSITION OF THE PRESENT INVENTION

The composition of the present invention was tested *in vivo* for anti-wrinkle efficacy and to assess improvements in skin smoothness and elasticity.

The test was conducted as a long-term study on 20 women over 28 days of daily application of the product to the face. A thin layer of the product was applied to previously cleansed skin until complete absorption, twice daily.

**The parameters were evaluated in the following areas:**
**For the cream:**
   **T0:** before treatment (baseline skin values),
   **T1:** after 7 days of treatment,
   **T2:** after 14 days of treatment,
   **T3:** after 28 days of treatment.
**For the serum:**
   **T0:** before treatment (baseline skin values),
   **T1:** after the first application (measurement after 15 minutes - only for visible wrinkles),
   **T1**: after 7 days of treatment,
   **T2**: after 14 days of treatment,
   **T3:** after 28 days of treatment.

The following tests were conducted:
For the serum and cream:
   i) Texture Analysis: using Antera 3D^{®} at each analysis time point (texture index).
   ii) Roughness Analysis: using Antera 3D^{®} at each analysis time point (average depth parameter)
   iii) Elasticity/Tone Analysis: using Cutometer^{®} only at T0 and T1.
Additionally, for the serum:
   iv) Wrinkle visibility analysis: using CameraScan^{®} (image acquisition) and Framescan^{®} (analysis software) by Orion Technologies only at T0 and T1 (visibility coefficient).

Antera 3D^{®}: This is a skin-scanning device that uses a specialized camera and is powered by sophisticated software called Antera 3D (Miravex, Ireland). The device consists of a camera for optical capture and software that uses complex algorithms to analyze the images in three dimensions. Antera 3D^{®} relies on multidirectional lighting and computer-aided reconstruction of the skin's surface, illuminating the surface from various angles and using the differences between these images to reconstruct the surface in three dimensions. The 3D skin topography and chromophore concentration are obtained through spatial and spectral analysis of the acquired image data, which is obtained by illuminating the skin surface from various directions using a specific multispectral LED system.

To measure the parameters of interest, a representative area is marked in the pre-treatment image; then, the same area is automatically captured in the follow-up image, and the parameter of interest is calculated.

Skin texture is determined by the Antera software by measuring the average roughness Ra using a roughness plane correction option. Roughness is quantified by the vertical deviations of a real surface from its ideal form. If these deviations are large, the surface is rough; if they are small, the surface is smooth.

Cutometer^{®}: is a system for diagnosing skin elasticity. The measurement is based on the principle of suction and stretching of the skin through a specific probe. A vacuum ranging from 20 to 500 mbar is generated at the probe tip of the instrument. The portion of skin being measured is drawn into the probe opening by the vacuum. The vacuum causes maximum deformation (known as extensibility), which decreases to a certain value when the vacuum is released. The difference between the extensibility value and the residual (plastic) deformation value is inversely proportional to the skin's elasticity. The instrument "reads" the deformation through an optical detection system located inside the probe, which sends the data to the microprocessor and from there, via a serial connection to the computer, to the application software. The instrument is capable of providing all the physical parameters relevant to the skin traction functions obtained using the suction method.

The instrument is capable of providing all physical parameters relevant to skin traction functions obtained using the suction method.
**Ue** = Elastic deformation of the skin following stress (suction);
**Uf** = Total maximum extensibility of the skin. Represents the passive resistance to skin deformation;
**Ur** = Elastic recovery upon removal of stress;
**Ua** = Complete recovery at the end of the deformation cycles;
**R2** = Ua / Uf = (Ua/Uf) the biological elasticity of the skin, including viscous deformation;
**R7** = Ur/Uf = Relative elastic recovery;
**R0** = Uf = Amplitude at the end of the suction phase of the first curve. This parameter reflects the skin's firmness/elasticity.

The results are expressed as the percentage increase in the relative elastic recovery (R7) and biological elasticity (R2) of Tn compared to the untreated sample (T0).

The term "Elasticity" refers to a material's property of spontaneously recovering its original shape and dimensions after the applied deforming force is removed.

Parameters related to elasticity are linked to skin aging and, in general, to the texture of the skin tissue itself. The greater the elasticity, the lower the risk of wrinkle development. Measuring parameters related to skin elasticity is useful for demonstrating the efficacy of products developed to combat skin aging.

Instrumental measurements are performed in a standardized manner and are conducted at 22°C ± 2°C and 50% RH.

### DATA ANALYSIS:

The statistical test applied is the t-test (α = 0.05). No statistical analysis is performed on the self-assessment.

### CREAM Results

| | | **Instrumental data** | | |
|---|---|---|---|---|
| | | **T1 vs T0** | **T2 vs TO** | **T3 vs T0** |
| **Texture** | **-** | **-3.9** | **-5.1** | **-7.7** |
| **Average wrinkle depth** | **-** | **-1.4** | **-2.3** | **.8.3** |
| **Elasticity** | **R0** | **-9.5** | **-** | **-** |
| | **R2** | **+12.5** | **-** | **-** |
| | **R7** | **+15.4** | **-** | **-** |

### SERUM Results

| | | **Instrumental data** | | |
|---|---|---|---|---|
| | | **T1 vs T0** | **T2 vs T0** | **T3 vs T0** |
| **Visibility of wrinkles** | | **-0.4** | | |
| **Texture** | | **-** | **-2.6** | **-6.3** |
| **Average wrinkle depth** | | **-** | **-1. 6** | **-2.9** |
| **Elasticity** | **R0** | **-** | **-41.6** | |
| | **R2** | **-** | **+7.4** | |
| | **R7** | **-** | **+13.3** | |

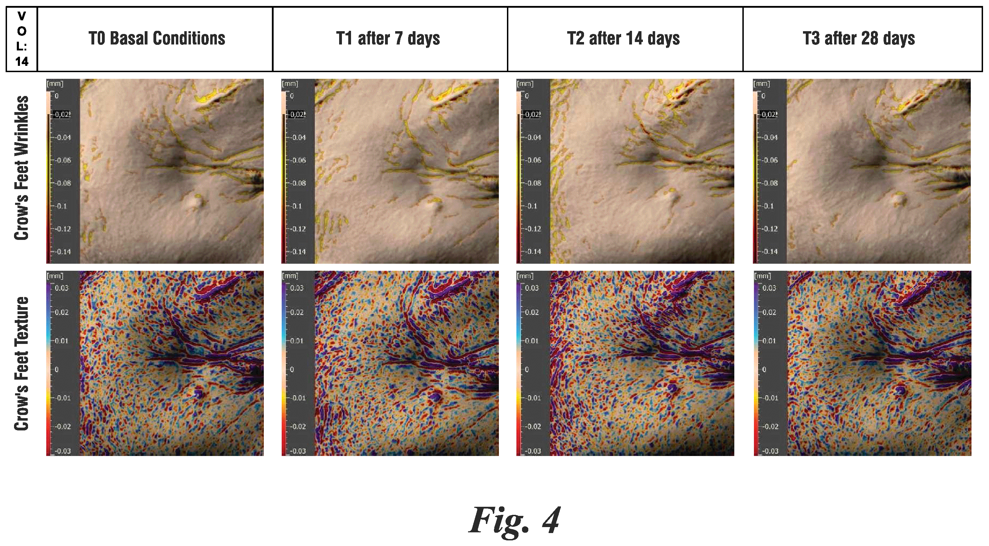

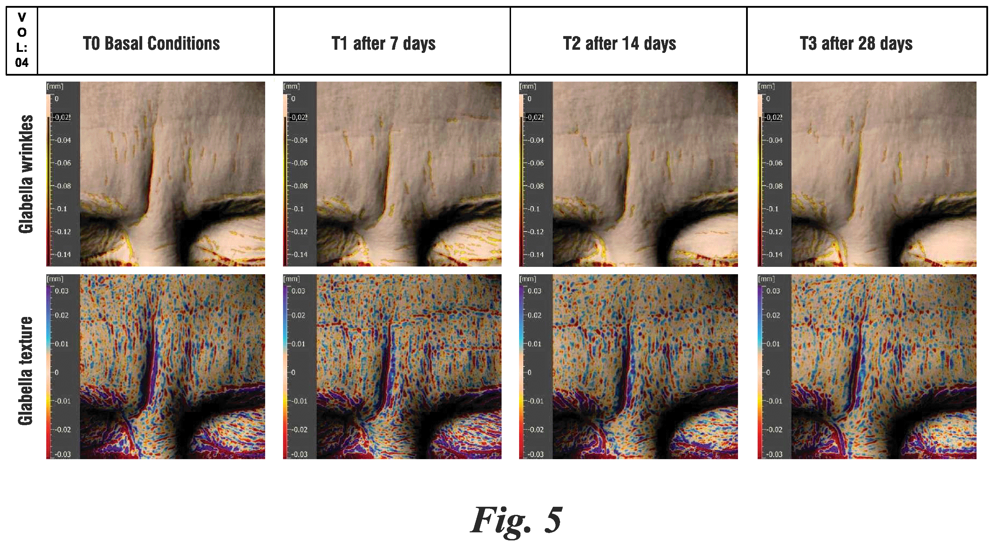

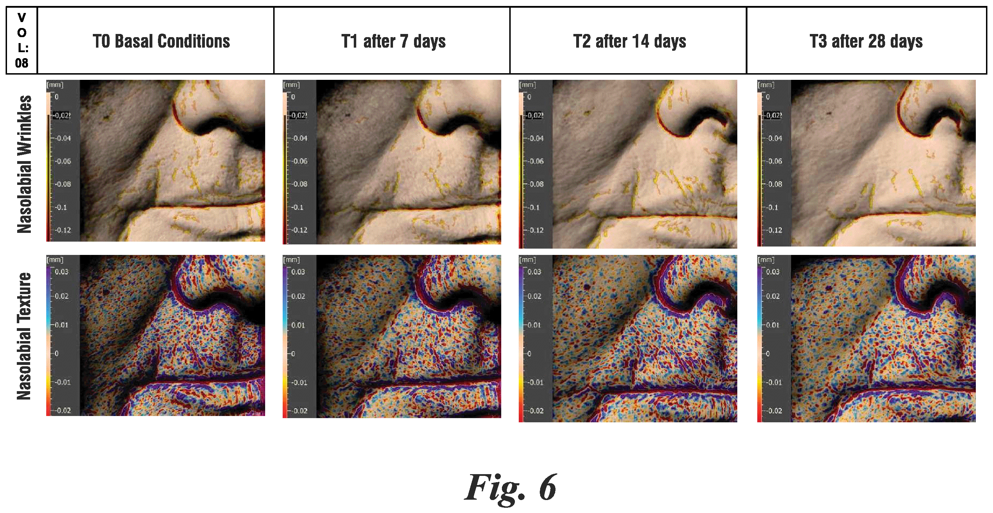

Figures 4, 5, and 6 show representative photos obtained using the Antera 3D instrument for the in vivo evaluation of the efficacy of the composition of the present invention in a cream formulation.

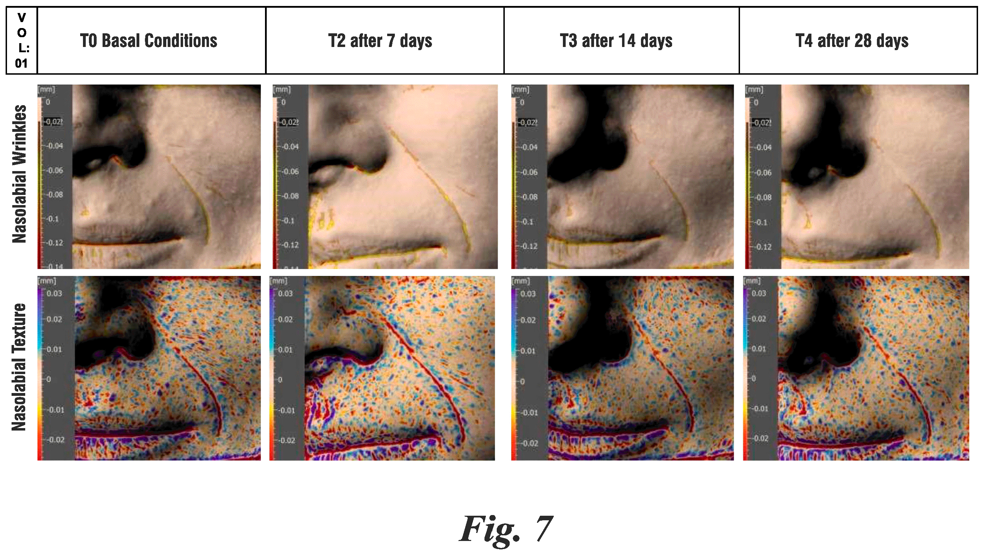

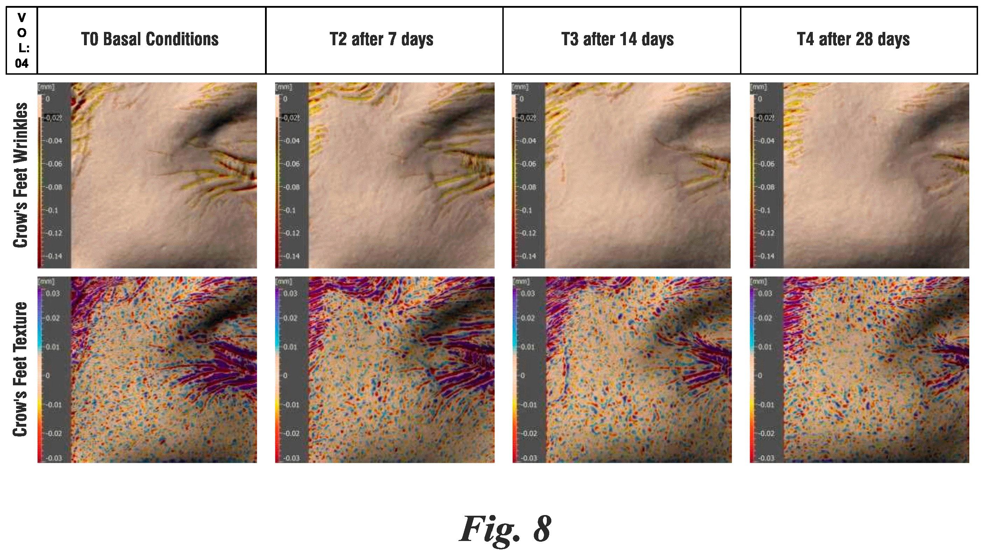

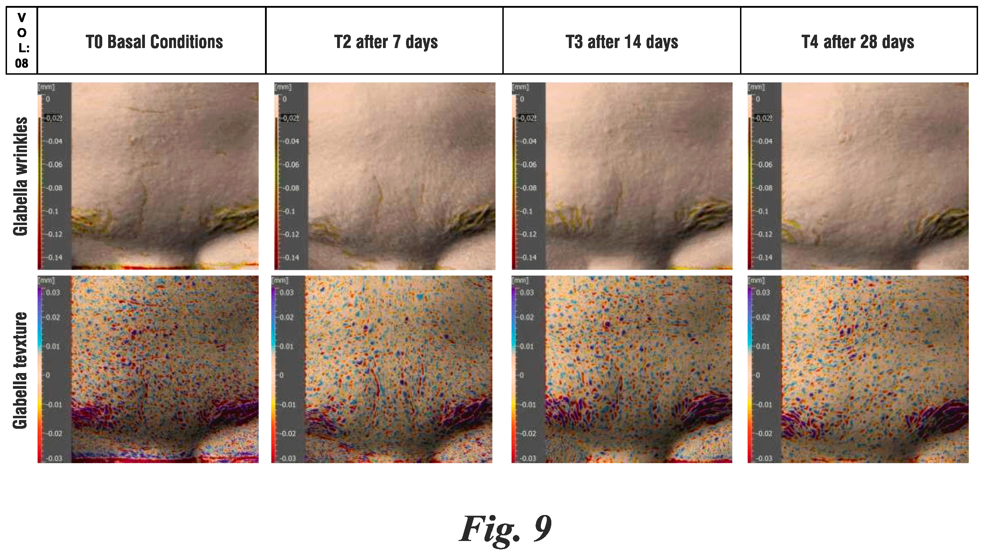

Figures 7, 8, and 9 show representative photos obtained using the Antera 3D instrument for the in vivo evaluation of the efficacy of the composition of the present invention in serum formulation.

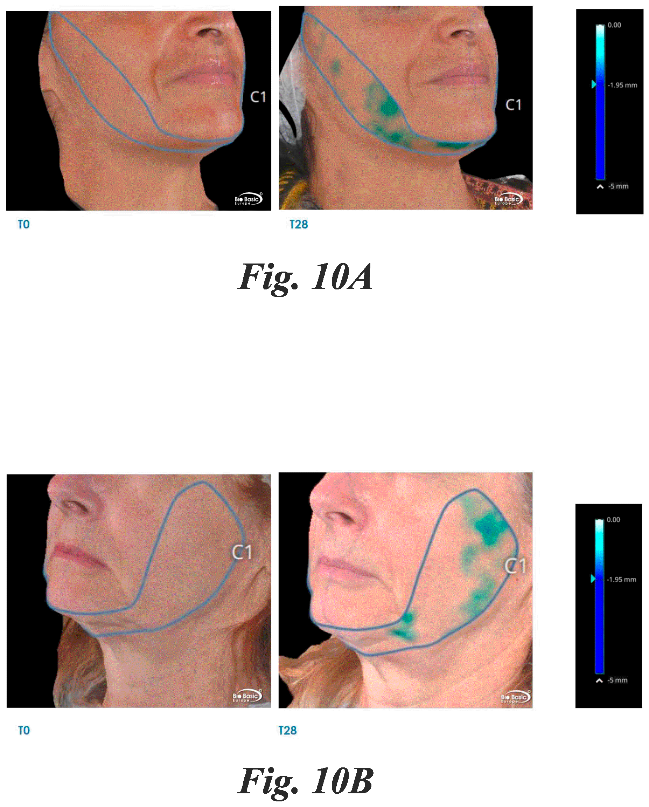

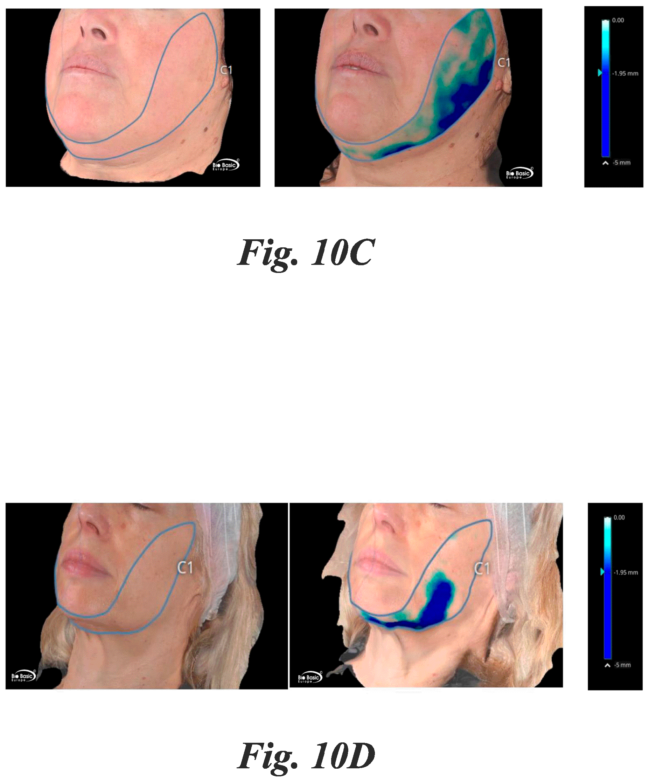

Figures 10A-D show photographs of the facial oval of subjects before and after treatment with cream (A, B) and serum (C, D).

The results obtained were also confirmed by subjective self-assessment tests conducted by the subjects participating in the experimental study.

### EXAMPLE 3

### IN VIVO EVALUATION OF THE EFFICACY OF A CLEANSER ON CELL RENEWAL AND SKIN RADIANCE

At the start of the study, each volunteer was given a bottle of cleanser containing the composition of the present invention, to be applied twice daily for 14 days.

The primary evaluation criterion considered was
- the evolution of cellular renewal, and
- skin radiance.

The primary assessment for the renewal effect was the measurement of color intensity in each area at different time points: T0, T6, and T12.

Three measurements were taken in each area, and the average value was calculated.

Brightness measurements were taken on the left cheek before (T0) and 7 days (T7) after product application. Measurements were taken 12 hours after the last application of the product.

### Method for assessing renewal

To determine the turnover time of the stratum corneum, dihydroxyacetone (DHA) was used as a marker; this substance selectively binds to corneocytes, producing a characteristic bronze coloration that can be measured by colorimetry.

The time it takes for the coloration to fade in the treated area compared to the untreated control area is easily standardized and represents the primary evaluation criterion for assessing cellular regeneration capacity.

For each volunteer, a 36 cm² area was marked on the inner side of the left forearm and anoth -sized area with the same characteristics on the inner side of the right forearm.

In these areas, a 5% DHA preparation was applied using a latex glove at a dose of 6 mg/cm² of body surface area (three times higher than that of a standard cream), left in contact for 15 minutes, and subsequently removed with a damp muslin cloth.

The product was applied to the test area according to two different protocols:
- Only 5% DHA was applied to the inner side of the right forearm as a control area. - 5% DHA plus the cleanser of the present invention was applied to the inner side of the left forearm twice daily for 12 days.

The product was applied under normal conditions of use, with a gentle massage.

Volunteers were asked not to use other cosmetic products in the application area during the study, but were allowed to use their usual personal hygiene products.

SkinColorCatch^{®} was used to determine the erythema and melanin indices insensitive to each color. It also displays the RGB, CIE L*a*b*, and L*c*h* color space coordinates and automatically calculates the ITA grade, which classifies skin tone.

Daylight-equivalent white LEDs are arranged in a circle inside the SkinColorCatch measurement chamber.

When the SkinColorCatch is gently placed on the skin, the LEDs illuminate it at a 45-degree angle to minimize skin gloss. The light reflected from the skin is detected by an RGB color sensor. The RGB and L*a*b* readings from the measurements are corrected using a reference color matrix measured with a spectrophotometer.

The measurement results show the erythema (E) and melanin (M) indices, which range from 0 to 999. The higher the reading, the greater the erythema/melanin in the skin. The screen displays the CIE L*a*b* color space coordinates, namely:
L: lightness ranging from black to white (0 → 100)
a: hue ranging from green to red (-128 → +127)
b: hue ranging from blue to yellow (-128 → +127)

The first screen also displays the ITA grade, which classifies skin tone. The classification of skin tone based on the ITA grade is presented in the table below.

| **ITA° range** | **Skin color classification** |
|---|---|
| 55-90 | Very light |
| 41-54 | Fair |
| 28-40 | Intermediate |
| 10-27 | Tanned |
| -30-9 | Brown |
| -90-29 | Dark |

To determine brightness, a Skinglossmeter^{®} was used, which measures light reflection on the skin by reflecting the light beam at the same angle as the contact surface. The instrument used a laser diode emitting a red-light beam at 635 nm as its light source. The diameter of the laser beam's point of impact on the skin surface is 50 microns.

Cosmetic acceptability was assessed using a subjective evaluation questionnaire, to be completed by each volunteer, which includes the product's sensory and subjectivity, as well as its tolerability.

After treatment, skin conditions will be assessed, specifically: more hydrated, softer, more comfortable, and more radiant skin. An overall evaluation of the product was also conducted.

Experimental conditions. Before taking measurements, volunteers rested for 20 minutes to acclimate to the experimental conditions (24-26 °C, 40-60% relative humidity), which were maintained constant throughout the procedure.

### RESULTS

The color differences observed between the control area and the treated areas were expressed as a percentage (%) relative to T0 and T24.

Increase in exfoliation - Cell renewal as a percentage:Δ RC (%) was calculated using the following formula:Δ RC (%) =Δ % C -Δ % T.

Δ% C: Reduction (%) in staining in the control area at various time points compared to T 24.

Δ% T: Reduction (%) in coloration in the treated area at different time points relative to T 24.

Individual results were expressed as absolute values for each experimental time point. The observed changes were expressed as percentages.

Statistical analysis of skin brightness measurements was performed using a Wilcoxon test for paired samples, with a significance level of p ≤ 0.05. The observed changes were expressed as a percentage relative to the control. Evolution of the stratum corneum turnover time Average melanin content obtained on the inner part of the right forearm: 5% DHA (CONTROL) compared to T 0 and T 24 and changes (%)

| **5% DHA (control)** | **T0** | **T24h** | **T6d** | **T12d** |
|---|---|---|---|---|
| **Mean (n=20)** | 538 | 580 | 561 | 547 |
| **Relative difference compared to T0** | - | 42 | 24 | 10 |
| **Relative change (%)** | - | +7.9 | +4.4 | +1.8 |

| **DHA 5% (control)** | **T24h** | **T6d** | **T12d** |
|---|---|---|---|
| **Mean (n=20)** | 580 | 561 | 547 |
| **Relative difference compared to T0** | - | -1.9 | -33 |
| **Relative change (%)** | - | -3.2 | -5.6 |

Average melanin level obtained on the inner left forearm: 5% DHA + detergent of the present invention applied twice daily for 12 days, relative to T0 and T 24h and corresponding changes (%)

| **DHA 5% + cleanser** | **T0** | **T24h** | **T6d** | **T12d** |
|---|---|---|---|---|
| **Average (n=20)** | 530 | 573 | 546 | 528 |
| **Relative difference between T and T0** | - | 43 | 16 | -3 |
| **Relative change (%)** | - | +8.0 | +3.0 | -0.5 |

| **DHA 5% + cleanser** | **T24h** | **T6d** | **T12d** |
|---|---|---|---|
| **Average (n=20)** | 573 | 546 | 528 |
| **Relative difference compared to T24** | - | -27 | -45 |
| **Relative change (%)** | - | -4.7 | -7.9 |

Increase in Exfoliation - Cell Renewal (%) obtained on the inner left forearm: 5% DHA + cleanser of the present invention twice daily, relative to T 24h and related changes (%).

| **DHA 5% + cleanser** | **T6d** | **T12d** |
|---|---|---|
| **Relative difference compared to the control area** | **+1.5** | **+2.3** |

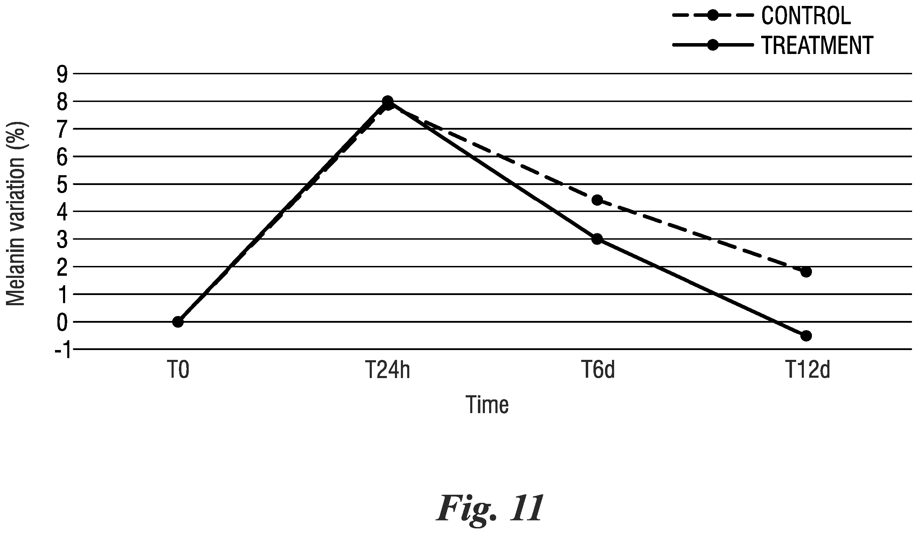

Figure 11 shows a graph depicting the percentage change in melatonin levels at various time points.

### Changes in skin brightness

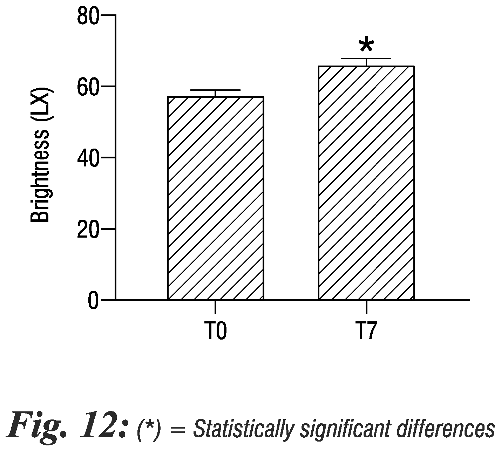

After 7 days of applying the cleanser of the present invention twice daily, the following brightness results (Skinglossmeter^{®}) were obtained, as shown in Figure 12.

| **Volunteers (n=20)** | **Mean values Δ%** | **Significance (p)** |
|---|---|---|
| **Brightness** | **+14.9** | **P<0.0001** |

The data obtained above were confirmed by a subjective self-assessment regarding the cosmetic quality of the products of the present invention.

After 12 days of daily application of 5% DHA + cleanser of the present invention (twice daily), an increase in exfoliation of +1.5 and +2.3, respectively, was observed compared to the same time period in the untreated control area.

This increase in exfoliation was also evident chronologically, as the color faded more rapidly in the treated area compared to the untreated control area.

The turnover time of the stratum corneum was therefore faster in the area treated with the cleanser of the present invention.

Furthermore, 7 days after applying the cleanser of the present invention twice daily, there was a significant increase in brightness of +14.9% (p < 0.0001).

Cosmetic acceptability was rated as very good, with an overall score of 7.4.

Tolerability was also excellent.

### EXAMPLE 4

### EXTRACTION PROCESS FROM LEAVES OF CENTELLA ASIATICA - PRODUCTION OF CENTELLA ASIATICA HETEROSIDES

The extraction process from leaves of Centella Asiatica has been performed using the protocol for IN-3022035 CENTEROX (Regulatory Affairs and Product safety - Givaudan France SAS, 55 Voie des Bans, 95100 Argenteuil, France).

The process is based on three phases:
i) production of precipitate 1: said phase comprising the following steps:
   - extraction with water/solvent from Centella asiatica leaves,
   - concentration / dilution,
   - liquid/liquid extraction,
   - concentration,
   - precipitation/centrifugation;
ii) Production of precipitate 2: said phase comprising the following steps:
   - Liquid/liquid extraction from the discarded materials;
   - Concentration,
   - -pH correction/extractions,
   - Extraction with solvent,
   - Crystallization;
iii) Production of Centella asiatica heterosides: said phase comprising the following steps:
   - Concentration of mother liquors and further precipitation,
   - Drying,
   - Packaging and quality control: approval of Centella asiatica heterosides intermediates,
   - Dissolution of precipitate 1 with precipitate 2,
   - Decoloration/concentration,
   - Drying,
   - Grinding,
   - Packaging/quality control/approval,
   - Final packaging/batch release of Centella asiatica heterosides.

## Claims

1. Use of a dermocosmetic composition in the field of dermocosmetics, wherein said dermocosmetic composition comprises the ingredients:
i) exosomes, preferably from Lactobacillus species;
ii) Centella asiatica extract, wherein said Centella asiatica extract is further supplemented with cyclic terpenes selected from the group consisting of: madecassoside, asiaticoside, and/or combinations thereof,
wherein said cyclic terpenes are obtained from Centella asiatica extract,
where said ingredients i) and ii) are present in a range of 0.0001-5% weight/weight (w/w) and said range relates to the amount of madecassoside, or asiaticoside, or to their combination alone, together with the amount of Centella asiatica extract already containing said compound inherently,
and
wherein said dermocosmetic use is to counteract cellular senescence.

2. Use of the dermocosmetic composition according to claim 1, wherein said ingredients i) and ii) are present in a range of 0.001-2% w/w.

3. Products for use and comprising the composition according to one or more of the claims, wherein said products are selected from the group consisting of: serum, cream, cleanser.
